# EUROPEAN PATENT APPLICATION

(11) **EP 4 512 337 A1**
(43) Date of publication of application: **26.02.2025**
(21) Application number: 23791958.4
(22) Date of filing: 21.04.2023
(51) Int. Cl.: A61B 5/346, A61B 5/339, A61B 5/358

(54) **ELECTROCARDIOGRAPH AND METHOD FOR CONTROLLING SAME**

(30) Priority: 21.04.2022 JP 2022070086
(71) Applicant: Fukuda Denshi Co., Ltd., Tokyo 113-8483 (JP)
(72) Inventor: KURIHARA, Setsuya, Tokyo 113-8483 (JP); HOSHINO, Masayuki, Tokyo 113-8483 (JP); DOYEN, Tyler, Tokyo 113-8483 (JP); DOYAMA, Taketo, Tokyo 113-8483 (JP); MIYAMA, Daichi, Tokyo 113-8483 (JP); GOTO, Takafumi, Tokyo 113-8483 (JP); JIMBA, Natsuko, Tokyo 113-8483 (JP)
(74) Representative: Mathys & Squire
(86) International application number: PCT/JP2023/015995
(87) International publication number: WO 2023/204310

(57) **Abstract**

Disclosed is an electrocardiograph and a method for controlling the electrocardiograph having a function that reduces a burden on medical stuffs who are involved in an electrocardiogram test and evaluation for evaluating the possibility of ACS. The electrocardiograph automatically applies analysis processing to an electrocardiogram while continuously obtaining an electrocardiogram of the subject and displaying the electrocardiogram. The electrocardiograph executes an enhanced test that includes periodical execution of an electrocardiogram test, if the information obtained through the analysis processing satisfies the condition regarding the possibility of acute coronary syndromes (ACS).

## Description

### TECHNICAL FIELD

The present invention relates to an electrocardiogram and a method for controlling the same.

### BACKGROUND ART

Acute coronary syndromes (ACS) are diseased states in which acute myocardial ischemia due to high-grade coronary stenosis or coronary occlusion occurs. In an acute coronary syndromes guideline (revised in 2018) (Non-Patent Literature 1, hereinafter simply referred to as "the guideline"), the followings are recommended as electrocardiogram tests for a subject for whomACS is suspected.
- Evaluate a 12-lead electrocardiogram (ECG) (first ECG) within 10 minutes.
- If it is not possible to make a diagnosis based on the first ECG, perform the followings according to symptoms or the like:
   record a 12-lead ECG every 5 to 10 minutes;
   record a 12-lead ECG over time;
   record lead V4R in addition to 12 leads; and
   consider recording leads V7 to V9.

### CITATION LIST

### NON-PATENT LITERATURE

NPL 1: Kimura et al., "Acute Coronary Syndromes Guideline (revised in 2018)", [online], issued on March 29, 2019, the Japanese Circulation Society, [searched on October 5, 2021], Internet <URL: https://www.j-circ.or.jp/cms/wp-content/uploads/2020/02/JCS2018_kimura.pdf>

### SUMMARY OF INVENTION

### TECHNICAL PROBLEM

However, in an emergency room where various treatments are performed in parallel on a subject who has been taken by an ambulance due to having a pain in the chest, for example, it is not easy to perform the ECG tests recommended in the guideline, in particular, the ECG test recommended when it is not possible to make a diagnosis based on the first ECG. Moreover, even if an ECG is taken, a doctor who can evaluate the ECG is not always present in the emergency room. Furthermore, when an ECG is taken multiple times, it is necessary to make a diagnosis by comparing multiple reports each output for every measurement, which is cumbersome.

For these reasons, there is a problem that a situation may occur in which evaluation regarding the possibility of ACS is not sufficiently performed particularly for a subject for whom it is not possible to make a diagnosis based on the first electrocardiogram.

In view of the above problems of conventional techniques, an aspect of the present invention provides an electrocardiograph having a function that reduces a burden on medical stuffs who are involved in an electrocardiogram test and evaluation for evaluating the possibility of ACS and a method for controlling the electrocardiograph.

### SOLUTION TO PROBLEM

According to an aspect of the present invention, there is provided an electrocardiograph comprising: obtaining means for continuously obtaining an electrocardiogram of a subject; display means for continuously displaying the electrocardiogram on a display device; analysis means for automatically applying analysis processing to the electrocardiogram in parallel with display of the electrocardiogram by the display means; and determination means for determining whether or not information obtained through the analysis processing satisfies a condition determined in advance regarding a possibility of acute coronary syndromes (ACS), the electrocardiograph further comprising control means for controlling the electrocardiograph to execute an enhanced test including periodical execution of an electrocardiogram test when it is determined by the determination means that the information obtained through the analysis processing satisfies the condition regarding the possibility of ACS before an ending condition determined in advance is satisfied.

### ADVANTAGEOUS EFFECTS OF INVENTION

According to the present invention, it is possible to provide an electrocardiograph having a function that reduces a burden on medical stuffs who are involved in an electrocardiogram test and evaluation for evaluating the possibility of ACS and a method for controlling the electrocardiograph.

### BRIEF DESCRIPTION OF DRAWINGS

The accompanying drawings, which are incorporated in and constitute a part of the specification, illustrate embodiments of the invention and, together with the description, serve to explain principles of the invention.
FIG. 1 is a block diagram showing a functional configuration example of an electrocardiograph according to an embodiment.
FIG. 2A is a flowchart relating to operations of the electrocardiograph according to an embodiment.
FIG. 2B is a flowchart relating to operations of the electrocardiograph according to an embodiment.
FIG. 3 is a diagram showing an example of a screen presented by the electrocardiograph according to an embodiment.
FIG. 4 is a diagram showing an example of a screen presented by the electrocardiograph according to an embodiment.
FIG. 5 is a diagram showing an example of a screen presented by the electrocardiograph according to an embodiment.
FIG. 6 is a diagram showing an example of a screen presented by the electrocardiograph according to an embodiment.
FIG. 7 is a diagram showing an example of a screen presented by the electrocardiograph according to an embodiment.
FIG. 8 is a diagram showing an example of a screen presented by the electrocardiograph according to an embodiment.
FIG. 9 is a diagram showing an example of a screen presented by the electrocardiograph according to an embodiment.
FIG. 10 is a diagram showing an example of a screen presented by the electrocardiograph according to an embodiment.
FIG. 11A is a diagram showing an example of a summary report output by the electrocardiograph according to an embodiment.
FIG. 11B is a diagram showing an example of a summary report output by the electrocardiograph according to an embodiment.
FIG. 12A is a diagram showing an example of a summary report output by the electrocardiograph according to an embodiment.
FIG. 12B is a diagram showing an example of a summary report output by the electrocardiograph according to an embodiment.
FIG. 13 is a diagram showing an example of a summary report output by the electrocardiograph according to an embodiment.

### DESCRIPTION OF EMBODIMENTS

Hereinafter, the present invention will be described in detail based on example embodiments thereof, with reference to the accompanying drawings. Note that the following embodiments do not limit the invention as set forth in the scope of patent claims. Additionally, although several features are described in the embodiments, all of these features are not necessarily required for the invention, and multiple features may be combined as desired. Furthermore, in the accompanying drawings, the same or similar configurations are given the same reference numerals, and redundant descriptions thereof will be omitted.

The following describes an embodiment in which the present invention is applied to an electrocardiograph, but the present invention is applicable to any other electronic device that can measure and analyze a standard 12-lead electrocardiogram (12-lead ECG). Such electronic devices include not only medical equipment such as a monitoring device but also a general-purpose computer device (a personal computer, a tablet terminal, a smartphone, etc.) that includes an interface to which electrocardiogram electrodes can be connected and that can execute an application for measuring and analyzing a standard 12-lead ECG.

### Configuration of Electrocardiograph

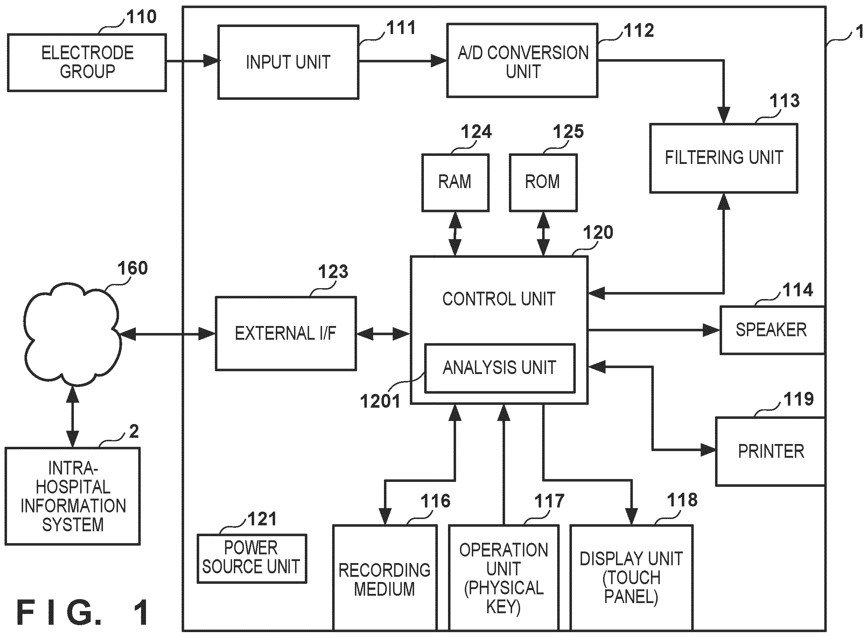

FIG. 1 is a block diagram showing a configuration example of an electrocardiograph 1 according to an embodiment of the present invention.

An electrode group 110 has a structure in which a plurality of electrodes that are to be attached to the body surface of a subject and a connector that is to be connected to an input unit 111 of the electrocardiograph are connected with lead wires. In the present embodiment, the electrocardiograph 1 measures a standard 12-lead ECG with use of the electrode group 110. The electrode group 110 used for the measurement of the standard 12-lead ECG generally includes (four) limb electrodes and (six) chest electrodes. Note that signals may also be input from the electrodes to the electrocardiograph via wireless communication rather than via the lead wires.

The input unit 111 includes a connector that fit with the connector included in the electrode group 110. The input unit 111 further includes a protective circuit, a lead selector, an amplification circuit, etc., for example, and outputs ECG signals of a type set in advance (here, ECG signals of the standard 12-lead ECG).

An A/D conversion unit 112 performs A/D conversion on analog ECG signals output from the input unit 111 and outputs ECG data. The input unit 111 and the A/D conversion unit 112 are also called an analog front end (AFE).

A filtering unit 113 applies a noise removal filter to the ECG data input from the A/D conversion unit 112 in accordance with a setting set by a control unit 120. In the present embodiment, the filtering unit 113 can selectively apply an electromyographic noise removal filter, an AC noise removal filter, and a drift noise removal filter, for example, but types and the number of filtering process are not limited to these examples.

A speaker 114 is used by the control unit 120 to output a warning sound or audio message, for example.

The control unit 120 (control means) is a processor that can execute programs, such as a CPU. The control unit 120 controls constituent elements of the electrocardiograph 1 and realizes operations of the electrocardiograph 1 including operations in an enhanced mode, which will be described later, by loading a program stored in a ROM 125 into a RAM 124 and executing the program with use of the processor.

In addition to the program, intrinsic information and setting values of the electrocardiograph 1, data of graphical user interfaces (GUIs) to be displayed in a display unit 118, and the like are stored in the ROM 125. The information and data stored in the ROM 125 are used as appropriate while the program is executed. Note that the ROM 125 may also be configured such that at least some information or data stored therein can be electrically rewritten.

The RAM 124 is used to load the program and store variables or the like while the program is executed. The RAM 124 is also used as a buffer memory and/or a video memory for the display unit 118.

Note that at least one of the A/D conversion unit 112 or the filtering unit 113 may be realized by the control unit 120 by executing the program or may be realized by a dedicated or existing hardware circuit.

An analysis unit 1201 (analysis means) applies analysis processing determined in advance to ECG data. The analysis unit 1201 represents, as a functional block, an analysis function of ECG data realized by the control unit 120 by executing the program. Accordingly, operations of the analysis unit 1201 are actually performed by the control unit 120. Note that the analysis unit 1201 may also be realized with use of a processor other than the control unit 120.

The analysis processing applied by the analysis unit 1201 is roughly divided into sectioning processing, feature value obtaining processing, and finding classification processing.

The sectioning processing is processing for obtaining ECG data of a predetermined unit (e.g., a predetermined number of heartbeats or a predetermined period of time) from measured ECG data.

The feature value obtaining processing is processing for detecting waveforms (e.g., P wave, QRS complex, T wave) constituting an ECG of a single heartbeat as well as dividing points among the waveforms, and for determining predetermined feature values (RR interval, ST level, PQ or PR interval, QRS width, QT interval, etc.).

The finding classification processing is processing for determining a plurality of findings including findings for which ACS is suspected, based on the feature values and predetermined determination conditions.

Note that the feature values listed above are merely examples, and other feature values may also be obtained. Also, there is no particular limitation on the findings to be determined. The analysis processing executed by the analysis unit 1201 is equivalent to processing realized as an automatic analysis function by a known electrocardiograph or a known ECG analysis device, and therefore, descriptions of further details of the analysis processing are omitted.

A recording medium 116 may be a removable medium such as a memory card or a USB memory, for example. The control unit 120 can record ECG test data in the recording medium 116 and read out ECG test data recorded in the recording medium 116. Note that ECG test data includes various types of data obtained through ECG measurement, such as subject information, ECG data, and analysis processing results. The ECG test data is generated in a predetermined data format for each test. Note that the ECG test data may be recorded as a plurality of data files associated with each other or a single data file.

An operation unit 117 is an input device that is used by a user (medical stuff) to input various settings and instructions to the electrocardiograph 1. In the present embodiment, the operation unit 117 includes a physical switch, a button, or a key provided on a housing of the electrocardiograph 1. The operation unit 117 includes a recording button for giving an instruction to start manual recording and an instruction for ending the manual recording.

The display unit 118 is a touch display including an LCD and a touch panel. The display unit 118 displays a measured ECG in real time and displays subject information, analysis processing results, various GUIs, etc., in accordance with control performed by the control unit 120.

A printer 119 is a thermal printer, for example, and includes a recording paper conveying mechanism, a recording head, and a recording head driving circuit, for example. The printer 119 outputs a measured ECG in real time and outputs a report including a measured ECG and a result of analysis of the ECG in accordance with control performed by the control unit 120. Note that it is not essential that the electrocardiograph according to the present invention includes the printer 119. The printer 119 may also be an external printer that is communicably connected to the electrocardiograph 1. That is to say, it is sufficient that the electrocardiograph 1 can use a built-in printer or an external printer.

A power source unit 121 is a secondary battery or a commercial power source (AC adapter), for example, and supplies power to the constituent elements of the electrocardiograph 1.

An external I/F 123 is a communication interface for communication with external devices and has a configuration in accordance with one or more known wired or wireless communication standards. For example, the external I/F 123 may be in accordance with one or more standards such as USB, 100BASE-T, wireless LAN, and Bluetooth (registered trademark).

Here, the external I/F 123 of the electrocardiograph 1 is connected to an intra-hospital information system 2 via a LAN 160 installed in the hospital. Accordingly, the electrocardiograph 1 can communicate with the intra-hospital information system 2 via the external I/F 123. The intra-hospital information system 2 manages personal information and medical information such as past test results and diagnosis results in association with subject IDs.

The electrocardiograph 1 according to the present embodiment has, as operation modes, a normal mode in which the electrocardiograph operates as a conventional desktop electrocardiograph and an enhanced mode in which the electrocardiograph provides a function that reduces a burden on medical stuffs who are involved in an ECG test for evaluating the possibility of ACS and evaluation of results of the ECG test. An operation mode in which the electrocardiograph operates when it is booted can be selected by the user on a setting screen, for example. Descriptions of operations in the normal mode are omitted, and the following describes operations in the enhanced mode.

### Operations in Enhanced mode

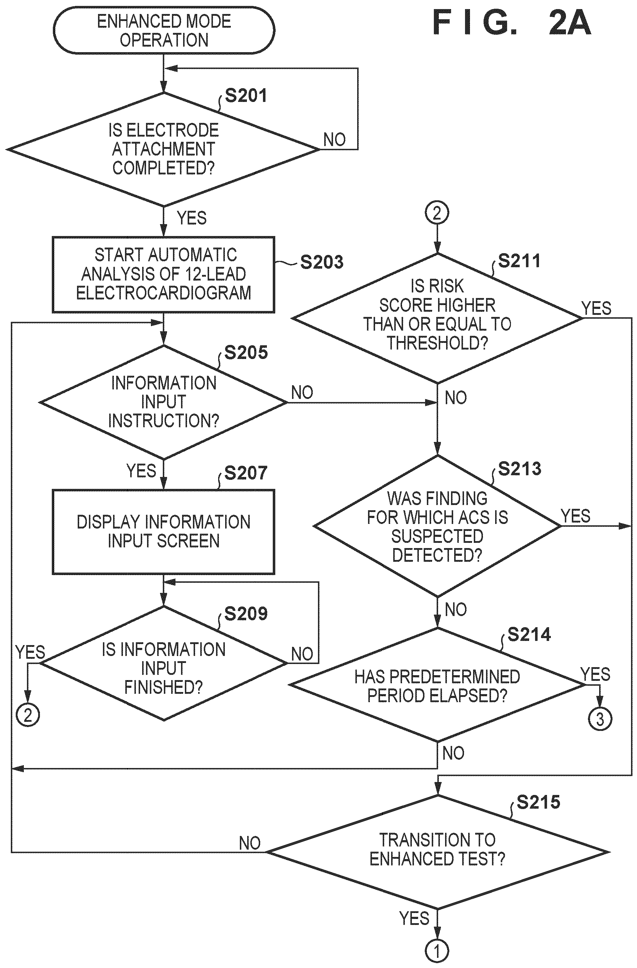

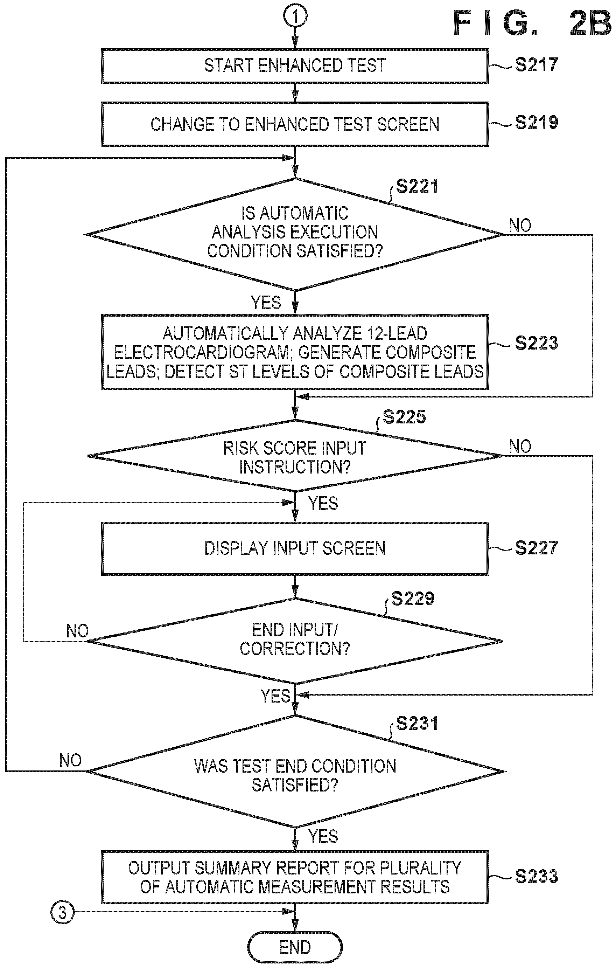

FIGS. 2A and 2B show a flowchart relating to operations of the electrocardiograph 1 in the enhanced mode. The operations shown in this flowchart are performed by the control unit 120 by loading the program stored in the ROM 125 into the RAM 124 and executing the program. Note that "an ECG" and "a lead" in the following description refer to those in states in which waveforms are visually recognizable (displayed or printed), and "ECG data" or " data of an ECG" and "lead data" or "data of a lead" respectively refer to data indicating the ECG and data indicating the lead. Note that "an ECG" includes one or more "leads".

The control unit 120 (obtaining means) continuously obtains an ECG of a subject based on signals input via the electrodes included in the electrode group 110 to the input unit 111. The control unit 120 (display means) continuously displays the obtained ECG in real time in the display unit 118. The control unit 120 sequentially starts real-time display from leads that can be displayed. Also, the control unit 120 executes the operations described hereinafter in parallel with the acquisition and real-time display of the ECG.

Furthermore, when an operation made on the operation unit 117 or a touch operation made on the display unit 118 is detected, the control unit 120 executes processing according to the detected operation in parallel with the operations described hereinafter. For example, when an operation is made on the recording button, the control unit 120 causes the printer 119 to output an ECG in real time as in the normal mode operations.

In step S201, the control unit 120 determines whether or not the electrodes included in the electrode group 110 have been attached to the subject, and upon determining that the electrodes have been attached, executes step S203, otherwise executes step S201 again. For example, when it is confirmed that data of all leads (here, leads I to III, lead aVR, lead aVL, lead aVF, and leads V1 to V6) that constitute an ECG to be recorded are obtained, the control unit 120 can determine that the electrodes have been attached.

In step S203, the control unit 120 starts to store ECG data in the RAM 124. The control unit 120 uses a predetermined region of the RAM 124 as a buffer (FIFO) for always keeping ECG data of the latest predetermined period of time (e.g., 30 seconds). Also, the control unit 120 starts to execute periodic analysis processing on the ECG data. These types of processing are automatically executed without any user instruction, and accordingly, evaluation of an ECG is executed in the background even when an instruction for recording (outputting) the first ECG is not given via the operation unit 117.

The control unit 120 (analysis unit 1201) periodically (e.g., in every 30 seconds) executes the analysis processing on ECG data of a predetermined period of time (e.g., 10 seconds). Note that, if the analysis processing can be executed in real time, it is also possible to execute the analysis processing on ECG data of the latest predetermined period of time.

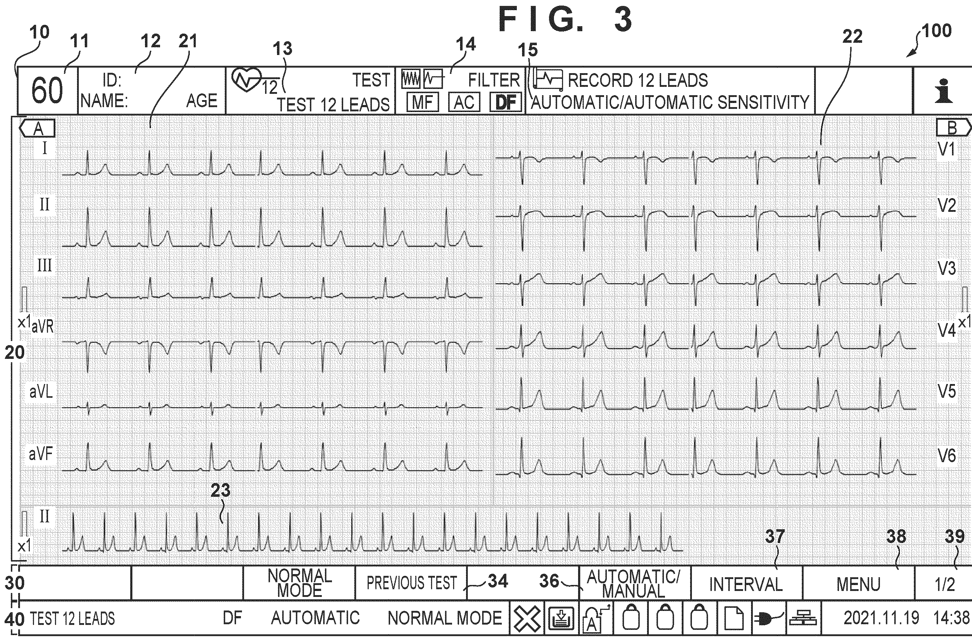

FIG. 3 shows an example of an initial screen of the enhanced mode, which is displayed in the display unit 118 by the control unit 120 when the electrodes have been attached and the periodic analysis processing is executed. The layout of the displayed screen and at least some of the displayed items can be changed by the user.

The initial screen 100 includes a waveform display region 20 in which a standard 12-lead ECG and one lead (here, lead II) determined in advance as a rhythm waveform from the standard 12 leads are displayed in real time. Also, a function key region 30 is provided under the waveform display region 20. Eight function keys that are GUI parts on which the user can make a touch operation are displayed in the function key region 30 in a switchable manner.

A first information display region 10 is provided above the waveform display region 20 (at the top of the initial screen 100), and a second information display region 40 is provided under the function key region 30 (at the bottom of the initial screen 100).

The following further describes each region. The first information display region 10 includes a heart rate region 11, a subject information region 12, a test type region 13, a filter type region 14, and a recording type region 15.

For example, an instantaneous heart rate (bpm) calculated from a time difference (e.g., RR interval) between feature points in an ECG of the latest two heartbeats is displayed in the heart rate region 11. Subject information such as identification information (subject ID), name, and age is displayed in the subject information region 12. A touch operation can be made on the subject information region 12, and the subject information can be input via a sub screen displayed in response to the touch operation (details will be described later).

A current test type is displayed in the test type region 13. The electrocardiograph 1 according to the present embodiment can carry out a 12-lead test and an 18-lead test in the enhanced mode, and a case where the 12-lead test is carried out in an initial state is shown in this example. Which of the 12-lead test and the 18-lead test is carried out when the electrocardiograph starts to operate in the enhanced mode can be set by the user. It is also possible to change the test type by touching the test type region 13.

Types of filters applied by the filtering unit 113 to the ECG data are displayed in the filter type region 14. As described above, the filtering unit 113 can selectively apply the electromyographic noise removal filter, the AC noise removal filter, and the drift noise removal filter. The user can change the types of filters applied by the filtering unit 113 by touching the filter type region 14.

The recording type region 15 displays either automatic recording or manual recording is executed in response to a recording instruction given via the recording button included in the operation unit 117 and recording settings such as the number of recorded leads. The automatic recording and the manual recording can be switched by making a touch operation on an automatic/manual recording switching key 36, which will be described later. Also, the recording settings can be set by the user via a menu screen.

The waveform display region 20 includes a region 21 for displaying limb leads (leads I to III and leads aVR, aVL, and aVF), a region 22 for displaying chest leads (leads V1 to V6), and a region 23 for displaying the rhythm waveform (here, lead II). The leads displayed in the waveform display region 20 are updated in real time, and leads of the latest predetermined period of time are always displayed. The rhythm waveform is displayed to evaluate regularity of heartbeats, and the waveform is compressed in the time direction to display a waveform of a period of time longer than the period of time displayed in the regions 21 and 22.

Types of the function keys displayed in the function key region 30 are determined according to the displayed screen. Also, at least some of the displayed function keys can be set by the user.

A previous test result call key 34 is a key for reading out information of ECG tests performed in the past on the subject whose ECG is being measured. When an operation is made on the previous test result call key 34, the control unit 120 makes an inquiry about results of past ECG tests to the intra-hospital information system 2 via the external I/F 123 based on the subject ID entered in the subject information region 12. When there are test results corresponding to the subject ID, the control unit 120 receives a list of the test results from the intra-hospital information system 2. The control unit 120 displays a test result selection screen based on the list in the display unit 118. Then, the control unit 120 obtains a test result selected in the selection screen from the intra-hospital information system 2 and causes the display unit 118 to display the test result.

When an operation is made on the automatic/manual recording switching key 36, the control unit 120 switches between an automatic recording operation and a manual recording operation, which are performed in response to an operation made on the recording button included in the operation unit 117. The currently set recording operation is displayed in the recording type region 15.

In the case of automatic recording, the control unit 120 causes the printer 119 to output an ECG of a certain period of time from when an operation is made on the recording button, as a report having a predetermined format together with a result of analysis processing performed by the analysis unit 1201. On the other hand, in the case of manual recording, when an operation is made on the recording button, the control unit 120 causes the printer 119 to continuously output a measured ECG in real time until an operation is again made on the recording button.

When an operation is made on an interval recording start key 37, the control unit 120 switches interval recording to effective or non-effective. When the interval recording is effective, the control unit 120 causes the printer 119 to output an ECG of a certain period of time every time a predetermined period of time elapses.

When an operation is made on a menu key 38, the control unit 120 causes the display unit 118 to display a menu screen. Note that the menu screen may be displayed as a sub window superimposed on the initial screen 100 or the initial screen 100 may be replaced with the menu screen.

When an operation is made on a page switching key 39, the control unit 120 switches the set of function keys displayed in the function key region 30. Here, a case where two sets of function keys can be switched is shown.

The second information display region 40 is a region for displaying current date and time, and settings and the state of the electrocardiograph 1, for example.

The following description refers to FIG. 2A again. While periodically executing the analysis processing, the control unit 120 determines in step S205 whether or not an information input instruction has been detected. The information input instruction is a touch operation made on the subject information region 12, for example. Upon determining that the information input instruction has been detected, the control unit 120 executes step S207, otherwise executes step S213.

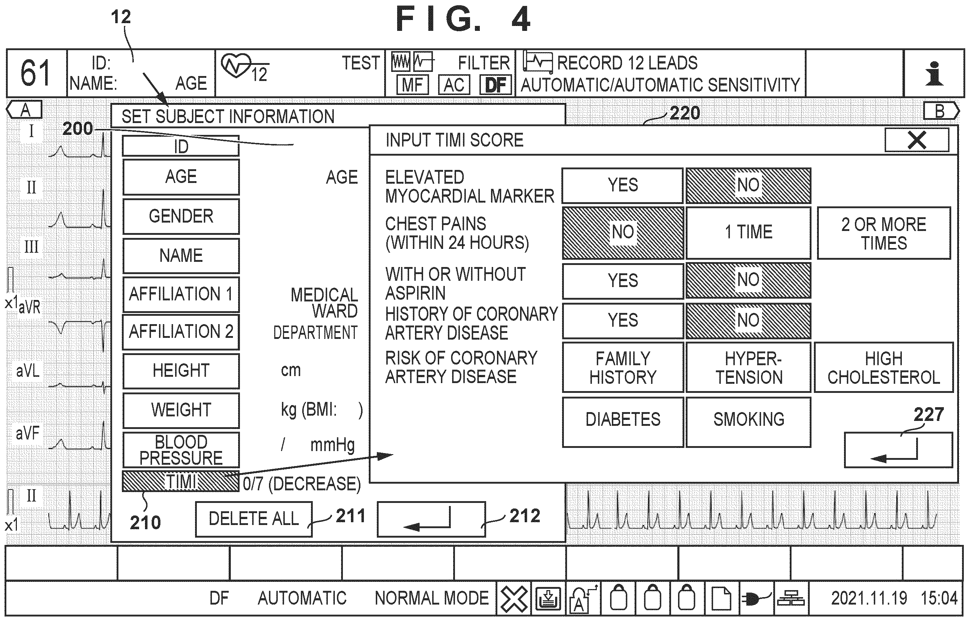

In step S207, the control unit 120 causes the display unit 118 to display an information input screen and executes step S209. FIG. 4 is a diagram showing an example of the input screen displayed in step S207. FIG. 4 shows an example in which the control unit 120 determines that a touch operation made on the subject information region 12 has been detected and displays a setting screen 200 for inputting subject information as a sub window superimposed on the initial screen 100.

The setting screen 200 includes buttons, on which a touch operation can be made, for respective items to be input. When an operation is made on a button, the control unit 120 activates a corresponding input region and further displays a software keyboard, an input screen, or the like according to the corresponding item. When an operation is made on an all delete button 211, the control unit 120 resets the setting screen 200 to an initial state. When an operation is made on a completion button 212, the control unit 120 closes the setting screen 200. Information that has been input to the setting screen 200 when an operation is made on the completion button 212 is stored in the ROM 125 or the recording medium 116 by the control unit 120. Note that it is sufficient to input only items that can be input among items of the subject information, and additional input or change can be made later.

Among the items to be input via the setting screen 200, "TIMI" represents a TIMI (Thrombolysis In Myocardial Infarction) risk score. The TIMI risk score is a numerical value indicating a risk of non-ST-elevation ACS (NSTE-ACS) and corresponds to the number of items that apply out of the following seven items.
(1) 65 years old or older.
(2) There is myocardial biomarker elevation.
(3) There were symptoms of angina pectoris twice or more in 24 hours.
(4) Aspirin was taken within 7 days.
(5) There is a past medical history of coronary artery disease with a stenosis percentage of 50% or more.
(6) Three or more of coronary risk factors (family history, high blood pressure, hypercholesterolemia, diabetes, currently smoking) apply.
(7) 0.5 mm or more ST displacement is observed in an ECG.

When an operation is made on a TIMI button 210 included in the setting screen 200, the control unit 120 further displays a TIMI score input screen 220 for inputting the items (2) to (6) out of the seven items listed above. Note that the item (1) is input in the setting screen 200 as an age item. Also, the item (7) is not input at this stage because analysis or evaluation of an ECG is necessary.

The TIMI score input screen 220 is configured such that it is possible to input the items (2) to (6) by touching buttons. Note that item names are simplified compared with the items included in the TIMI risk score because of limitation on the number of characters, for example. In the example shown in FIG. 4, input regarding the item (3) is selected from three options: "none", "once", and "twice or more". However, "none" and "once" are not distinguished in the TIMI risk score, and therefore, input regarding the item (3) may be selected from "less than twice" and "twice or more". Note that it is sufficient to input only items that can be input among the items of the TIMI score, and additional input or change can be made later.

When an operation is made on a completion button 227 displayed on the TIMI score input screen 220, the control unit 120 closes the TIMI score input screen 220. The control unit 120 calculates the TIMI score based on the states of the buttons included in the TIMI score input screen 220 at the time when the operation is made on the completion button 227, and inputs the TIMI score in a TIMI score region included in the setting screen 200.

When an operation is made on the completion button 212 included in the setting screen 200, the states of the buttons included in the TIMI score input screen 220 at the time when the operation is made on the completion button 227 are stored in the ROM 125 or the recording medium 116 by the control unit 120 similarly to the other subject information.

Note that the TIMI risk score is an example of an ACS risk score, and it is also possible to use another known ACS risk score such as the Global Registry of Acute Coronary Events (GRACE) risk score.

In step S209, the control unit 120 determines whether or not input of information has been finished. In the example shown in FIG. 4, the control unit 120 can determine that input of information has been finished, upon detecting an operation made on the completion button 212 included in the setting screen 200. Upon determining that input of information has been finished, the control unit 120 executes step S211 after storing subject information that has been input, otherwise repeatedly executes step S209.

In step S211, the control unit 120 (determination means) determines whether or not the risk score is higher than or equal to a threshold by referring to the input subject information. Here, the TIMI risk score is used as the ACS risk score, and the threshold is 1. That is to say, if at least one of the items (1) to (6) listed above applies, the control unit 120 determines that the risk score is higher than or equal to the threshold. Note that the threshold is determined according to the type of the ACS risk score that is used. A configuration is also possible in which the user can change the threshold. Upon determining that the risk score is higher than or equal to the threshold, the control unit 120 executes step S215, otherwise executes step S213.

In step S213, the control unit 120 (determination means) determines whether or not a finding for which ACS is suspected has been detected in the analysis processing started by the analysis unit 1201 from step S209. Examples of findings for which ACS is suspected include a finding that there is a suspicion or possibility of infarction and findings of elevation of an ST level (ST segment is higher than a baseline), T wave inversion (change from up-convexity form to down-convexity form), abnormal Q wave, increase in the QT interval, atrioventricular block, and the like. These findings can be detected through known analysis processing, and therefore, detailed descriptions thereof are omitted.

Upon determining that a finding for which ACS is suspected has been detected in the analysis processing, the control unit 120 executes step S215, otherwise executes step S214. Note that FIG. 2A shows step S213 as a processing step that is executed in the chronological order, but the analysis processing is executed in parallel with the processing performed in steps S205 to S211. Therefore, the control unit 120 actually executes step S213 every time the analysis processing is complete, and immediately executes step S215 upon determining that a finding for which ACS is suspected has been detected. Note that, when the information input screen is displayed, the control unit 120 may compulsorily end information input processing after notifying the user.

In step S214, the control unit 120 determines whether or not a predetermined period has elapsed from when the analysis processing was started in step S203, and upon determining that the predetermined period has elapsed, ends the operations in the enhanced mode, otherwise repeatedly executes the processing from step S205. Note that even after the operations in the enhanced mode are ended, measurement and real-time display of the ECG are continued until the electrodes are removed or the power source is turned off. The predetermined period is a period for determining that there is no suspicion of ACS and can be set to 1 hour, for example.

Note that, if it is determined in step S214 that a finding for which ACS is suspected has not been detected for the predetermined consecutive period, it is also possible to ask the user whether or not to continue the operations in the enhanced mode (here, execution of the periodic analysis processing). If the user gives an instruction to continue the operations, the control unit 120 resets the elapsed time to 0 and continues to execute the processing from step S205.

In step S215, the control unit 120 notifies the user of the fact that the risk score is higher than or equal to the threshold or a finding for which ACS is suspected has been detected in the analysis processing. Also, the control unit 120 determines whether or not to transition to an enhanced test.

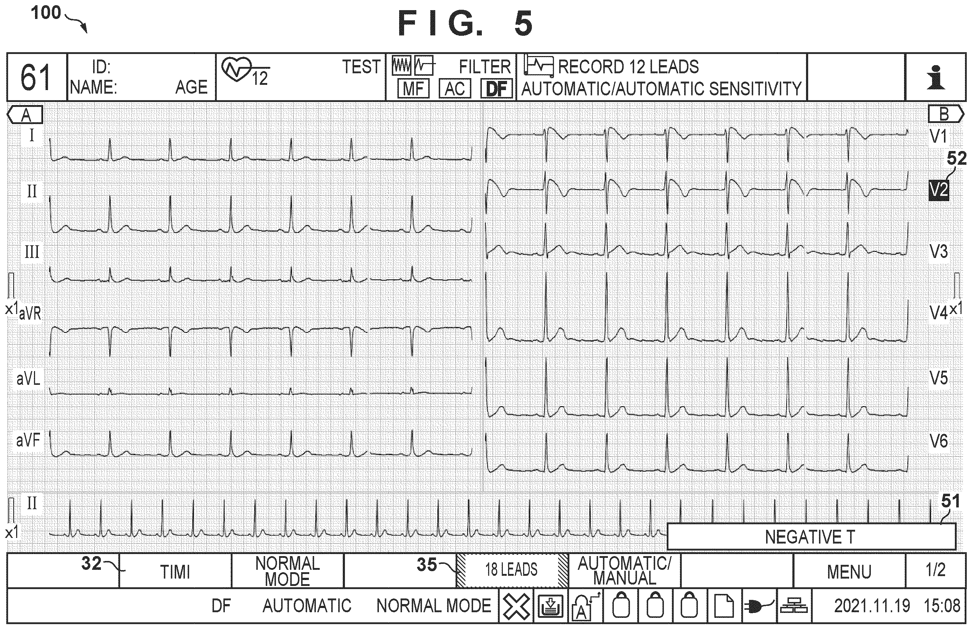

FIG. 5 is a diagram showing an example of display on the initial screen 100 when it is determined that a finding for which ACS is suspected has been detected in the analysis processing. The control unit 120 superimposes a message 51 indicating the name of the detected finding on the initial screen 100. Here, the background color of the message 51 is changed according to the importance (seriousness) of the finding. The importance of the finding is set in advance. Also, the control unit 120 notifies the user of a lead for which the finding indicated by the message 51 has been detected by highlighting an index 52 of the lead. Note that it is also possible to output a warning sound or audio from the speaker 114 in addition to the display described above.

As described above, the electrocardiograph 1 according to the present embodiment periodically executes analysis processing in parallel with real-time display of an ECG even if an explicit instruction is not given from the user, and if a finding for which ACS is suspected has been detected, notifies the user of the detected finding. Therefore, even in a case where it takes time until the first ECG is recorded, it is possible to detect a suspicion of ACS early. Also, even under situations in which a staff or a doctor who is skilled in reading an ECG is absent, it is possible to objectively grasp the suspicion of ACS by using the automatic analysis function of the electrocardiograph 1.

Furthermore, the control unit 120 changes the function keys displayed in the function key region 30. Here, a TIMI key 32 and an 18-lead key 35 are additionally displayed. The TIMI key 32 is used to input an item that has not been input or change an already input item among the items included in the TIMI risk score. For example, whether or not there is elevation of the myocardial marker may be unknown when the TIMI risk score is initially input, because a test is started after the subject is carried to the hospital. Accordingly, if a test result is available at this point in time, it is possible to additionally input the result by operating the TIMI key 32. Additional input can be performed not only for the elevation of the myocardial marker but also for other items that have not been input. Also, when there is an error in already input information, it is possible to correct the error at this time. Note that additional input or change may also be performed by making an operation on the subject information region 12 as described above.

The 18-lead key 35 is a key for inputting an instruction to execute the enhanced test. The control unit 120 waits for an operation being made on the 18-lead key 35, while displaying the standard 12-lead ECG in real time. Here, the 18-lead key 35 is displayed differently from the other function keys to make it easy for the user to recognize the operation required to input an instruction to transition to the enhanced test. In this example, the standard 12-lead ECG is measured in the initial state of the operations in the enhanced mode, and accordingly, an operation made on the 18-lead key 35 is taken to be the instruction to transition to the enhanced test. However, if an 18-lead ECG is measured in the initial state, it is also possible to display a function key showing "execute enhanced test", for example.

In this example, the user gives an explicit instruction regarding whether or not to execute the enhanced test, but a configuration is also possible in which the electrocardiograph automatically transitions to the enhanced test unless an operation for rejecting execution of the enhanced test (e.g., a predetermined operation made on the operation unit 117) is detected. For example, if a user operation instructing not to execute the enhanced test is not detected within a certain period of time after the message 51 is displayed, the control unit 120 may automatically execute step S217. Alternatively, it is also possible to automatically execute the enhanced test without asking the user whether or not to execute the enhanced test, with a notification of the finding as well as a start of the enhanced test using the message 51.

In step S217, the control unit 120 starts the enhanced test. Specifically, the enhanced test includes processing for
generating data of a right chest lead (at least lead V4R) and posterior leads (leads V7 to V9) through composite processing based on data of an actually measured standard 12-lead ECG,
periodically executing analysis processing (ECG test) on the data of the actually measured standard 12-lead ECG,
periodically detecting ST levels for data of the composite leads,
displaying a representative waveform and an ST level of each of 18 leads, and
outputting a single sheet of report collectively showing results of the analysis processing performed a plurality of times.

The following describes operations relating to the enhanced test.

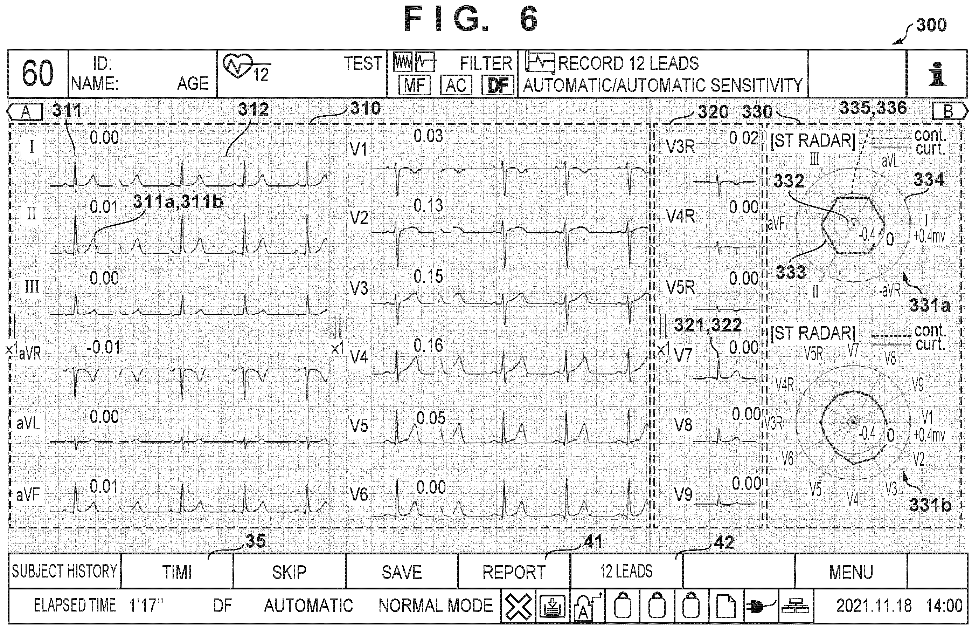

In step S219, the control unit 120 changes the displayed screen from the initial screen 100 to an enhanced test screen. FIG. 6 shows an example of the enhanced test screen 300. In the enhanced test screen 300, a region corresponding to the waveform display region 20 of the initial screen 100 includes a measured waveform display region 310 (first display region), a composite waveform display region 320 (second display region), and an ST level display region 330 (third display region).

The measured waveform display region 310 is a region for displaying a standard 12-lead ECG that is being measured by the electrocardiograph 1. Unlike the waveform display region 20 of the initial screen 100, the measured waveform display region 310 includes a representative waveform display region 311 in addition to a real-time display region 312. The real-time display region 312 is a region for displaying the measured standard 12-lead ECG in real time similarly to the waveform display region 20 of the initial screen 100.

The representative waveform display region 311 is a region for displaying a representative waveform of each lead. The representative waveform is a waveform of a single heartbeat representing an ECG of a predetermined period of time (e.g., 10 seconds) to which analysis processing has been applied. There is no particular limitation on the method for determining the representative waveform among waveforms of a plurality of heartbeats included in the ECG of the predetermined period of time, and a known method may be used. For example, the waveform of a single heartbeat that has the smallest difference from an average waveform (the highest correlation with the average waveform) may be taken to be the representative waveform.

Note that, in the present embodiment, a representative waveform in the standard 12-lead ECG of a predetermined period of time, which is used in analysis processing executed immediately before or after step S217 (the start of the enhanced test), is initially displayed as a control waveform (reference waveform) 311a in the representative waveform display region 311. Thereafter, every time analysis processing is executed in the enhanced test, a representative waveform 311b in the standard 12-lead ECG of the predetermined period of time used in the latest analysis processing is superimposed on the control waveform 311a. The superimposed latest representative waveform 311b is displayed differently (e.g., in a color different) from the control waveform 311a to make it easy to grasp a change between the waveform measured at the start of the enhanced test and the waveform measured later.

The composite waveform display region 320 is a region for displaying representative waveforms of leads obtained through composite processing. The composite processing is processing for generating data of leads that are not measured, based on data of actually measured leads. The composite processing makes it possible to obtain data of leads (composite lead data) that would be measured at positions on the body surface to which electrodes are not attached.

The composite processing makes it possible to obtain data of leads more than the standard 12 leads, and therefore, it is possible to provide information regarding leads that are useful in making a diagnosis on the possibility of ACS without increasing the number of electrodes attached to the subject.

In the present embodiment, ECG data of leads V3R to V5R and V7 to V9 including the right chest lead (lead V4R) and the posterior leads (leads V7 to V9) recommended in the guideline is generated through the composite processing based on data of the standard 12-lead ECG that is being measured.

A known method may be used to perform the composite processing for generating data of the leads V3R to V5R and V7 to V9, for which electrodes are not actually attached, from the data of the standard 12-lead ECG. For example, the control unit 120 generates data of leads X, Y, and Z from measured data of the standard 12-lead ECG by using the inverse Dower matrix. Then, the control unit 120 calculates an inner product of the data of the leads X, Y, Z and x, y, and z components of a lead vector of a virtual electrode position for each sample of lead data, and thus generates composite lead data of the virtual electrode position. Specific examples of the composite processing are described in Japanese Patent No. 4664068 and Japanese Patent No. 4955153, for example.

The control unit 120 generates data of the leads V3R to V5R and V7 to V9 of the predetermined period of time by performing composite processing using data of the standard 12-lead ECG of the predetermined period of time to which analysis processing is applied. Then, the control unit 120 determines a representative waveform of each lead obtained through the composite processing and displays the determined representative waveform in the composite waveform display region 320. The representative waveform may be determined using the same method as the method for determining representative waveforms of the standard 12 leads. Alternatively, the control unit 120 may display representative waveforms of the composite leads by using data of the leads V3R to V5R and V7 to V9 of a single heartbeat generated by applying the composite processing to data of the representative waveforms in the standard 12-lead ECG.

Also, the control unit 120 displays a representative waveform relating to the first composite processing as a control waveform 321 in the composite waveform display region 320, and as for the second or following composite processing, superimposes a representative waveform 322 relating to the latest composite processing on the control waveform. The latest representative waveform 322 is displayed differently from the control waveform 321 so that the control waveform and the latest representative waveform can be distinguished from each other.

The ST level display region 330 is a region for displaying an ST level for each of the standard 12 leads and the composite leads. Here, the ST level is a relative value when a baseline level is taken to be 0. In FIG. 6, the maximum ST level corresponding to the maximum amplitude in an ST segment is displayed as the ST level. For example, a straight line connecting a Q wave start point and a T wave end point of a heartbeat including the target ST segment can be used as the baseline, and an ST level that has the largest absolute value of a difference from the corresponding baseline level among sample values of the ST segment can be determined as the maximum ST level. The maximum ST level is an example of a representative value of the ST segment based on amplitude.

In the present embodiment, the 18 leads are separated into 6 limb leads (I, II, III, aVL, aVR, and aVF) and 12 chest leads (V1 to V6, V3R, V4R, V5R, and V7 to V9), and ST levels of the leads are displayed for each of the two types of leads. FIG. 6 shows an example in which the ST levels of the leads are displayed by plotting maximum ST levels on separate radar charts 331a and 331b corresponding to the respective types of leads.

Here, the maximum ST level detected in ECG data of the 18 leads (measured standard 12 leads and 6 composite leads) of the predetermined period of time to which analysis processing is applied is displayed with respect to each of the leads. Furthermore, in the present embodiment, the maximum ST level (control value) detected in analysis processing executed immediately before or after the start of the enhanced test (first analysis processing in the enhanced test) and the maximum ST level detected in the latest analysis processing in the enhanced test are displayed (plotted on the radar chart) with respect to each lead in the ST level display region 330 in such a manner that these maximum ST levels can be compared with each other. When the ECG data of the predetermined period of time includes ECG data of two or more heartbeats, the maximum ST level in all of the heartbeats is displayed.

The radar chart 331a shows maximum ST levels of the 6 limb leads. The radar chart 331a includes three concentric circles 332 to 334, and the circle 333 indicates an ST level of 0. Negative ST levels are shown on the center side relative to the circle 333, and positive ST levels are shown on the outer circumferential side relative to the circle 333. As for each axis, a distance between a point of intersection with the circle 333 and a point of intersection with the circle 334 is equal to a distance between the point of intersection with the circle 333 and a point of intersection with the circle 332. The radar chart 331b is the same as the radar chart 331a other than the types and the number of plotted leads.

Scales of the axes of the radar charts 331a and 331b are dynamically determined according to the absolute values of maximum ST levels of the 18 leads. Specifically, if the maximum ST levels are positive values, the scale of each axis is determined such that the largest absolute value is plotted on the circle 334. On the other hand, if the maximum ST levels are negative values, the scale of each axis is determined such that the largest absolute value is plotted on the circle 332. The axes of the radar charts 331a and 331b have the same scale.

A label showing the name of a lead is given to each axis of the radar charts 331a and 331b. As for the standard 12 leads, maximum ST levels 335 detected in the analysis processing executed immediately before or after the start of the enhanced test are plotted as control values (reference values) on the radar charts 331a and 331b. On the other hand, as for the 6 leads generated through the composite processing, maximum ST levels 335 detected in the analysis processing executed immediately after the start of the enhanced test (first analysis processing in the enhanced test) are plotted as control values (reference values) on the radar charts 331a and 331b. Also, as for all the 18 leads, maximum ST levels 336 (latest values) detected in the latest analysis processing, which is executed after the control values are obtained, are plotted on the radar charts 331a and 331b in such a manner that the maximum ST levels 336 can be visually distinguished from the control values. In FIG. 6, the control values (cont.) are shown by dotted lines, and the latest values (curt.) are shown by solid lines.

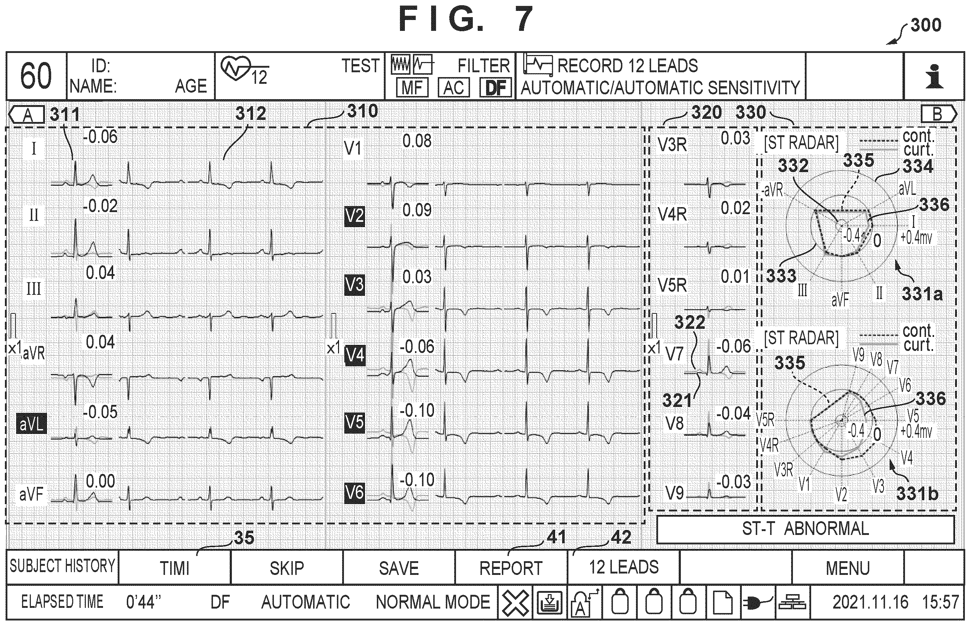

FIG. 7 shows a variation of the enhanced test screen 300 that is different from the enhanced test screen 300 shown in FIG. 6 in the ST level display region 330, or more specifically, the arrangement of the axes. In the example shown in FIG. 6, the axes respectively corresponding to the leads are arranged at equal intervals, but in this variation, the axes are arranged according to a positional relationship that simulates a positional relationship between the electrodes respectively corresponding to the leads on the body surface.

That is to say, in the radar chart 331a of the 6 limb leads, the axes corresponding to the leads are arranged in such a manner as to simulate positions on the limbs to which the electrodes are attached in a standing position, and in the radar chart 331b of the 12 chest leads, the axes corresponding to the leads are arranged in such a manner as to simulate positions on the chest surface to which the electrodes are attached.

The method for plotting the maximum ST levels on the radar charts 331a and 331b is the same as that used in the example shown in FIG. 6, and therefore, descriptions thereof are omitted. Also, display in regions other than the ST level display region 330 is the same as that in the example shown in FIG. 6, and therefore, descriptions thereof are omitted.

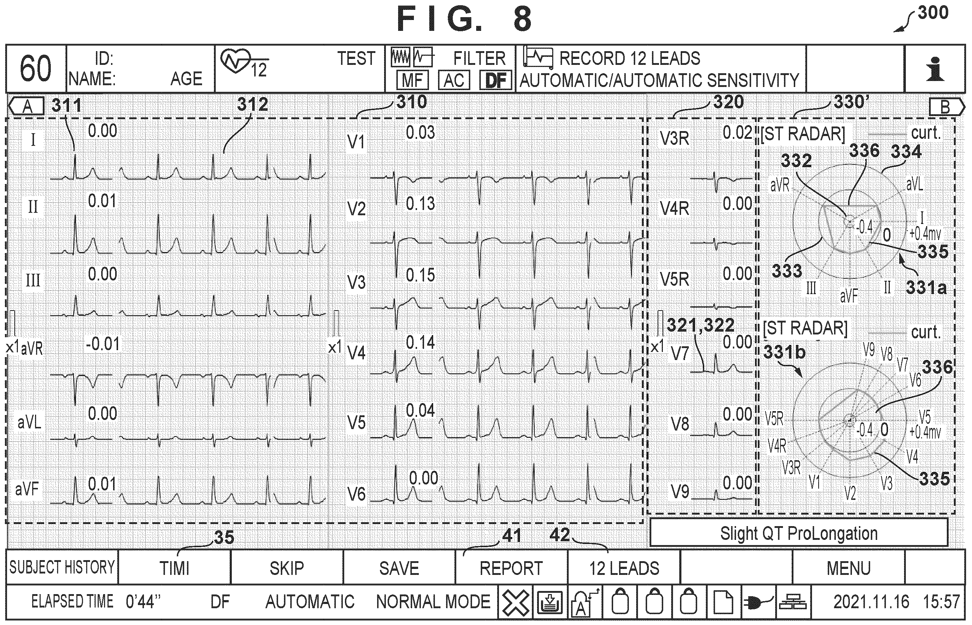

Note that another ST level may also be plotted on the radar charts, instead of the maximum ST level. FIG. 8 shows another variation of the enhanced test screen 300, which presents an ST level at a specific timing on the radar charts, instead of the maximum ST level. This enhanced test screen is the same as those in the examples shown in FIGS. 6 and 7 other than an ST level display region 330'. The ST level displayed in the example shown in FIG. 8 is an example of a representative value of an ST segment based on a timing.

In the example shown in FIG. 8, for example, an ST level corresponding to the amplitude at a timing when a predetermined period has elapsed from the start of the ST segment is displayed as an ST level corresponding to the amplitude at a specific timing in the ST segment determined in advance. The predetermined period may be an absolute value (fixed value) or a value that is dynamically determined based on a factor other than the ST level. An example of the value dynamically determined based on a factor other than the ST value may be a value that is determined according to the QT interval of a heartbeat including the target ST segment (e.g., a predetermined ratio such as 1/10 or 1/5 of the QT interval).

In the example shown in FIG. 8, ST levels detected in the latest analysis processing are displayed for the respective leads. Note that, when ECG data of the predetermined period of time to which the analysis processing is applied includes ECG data of two or more heartbeats, a representative value (e.g., an average value or the largest value) of a plurality of detected ST levels is displayed. Similarly to the examples shown in FIGS. 6 and 7, ST levels detected in the first analysis processing may also be displayed as control values in such a manner that the ST levels detected in the latest analysis processing can be compared with the control values.

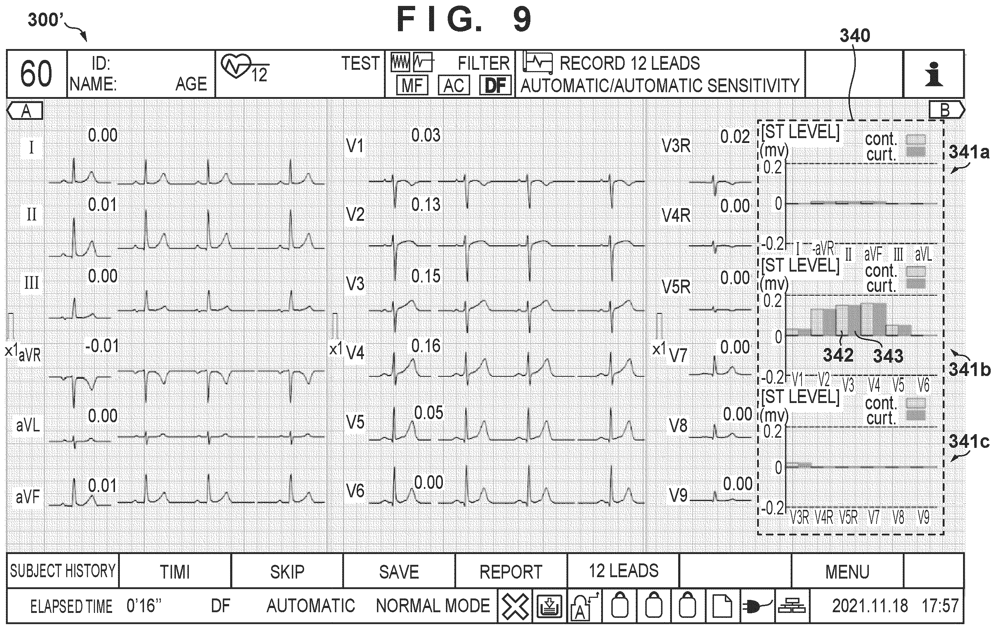

Note that ST levels may also be displayed by means other than radar charts. FIG. 9 shows an example of the enhanced test screen 300' including an ST level display region 340 in which radar charts are not used. In the ST level display region 340, ST levels are shown by bar graphs 341a to 341c.

The bar graph 341a shows ST levels of the 6 limb leads. The bar graphs 341b and 341c respectively show ST levels of the actually measured 6 leads (V 1 to V6) and ST levels of the 6 composite leads (V3R to V5R and V7 to V9) of the 12 chest leads. In the case where the bar graphs are used, a control value 342 and the latest value 343 of each lead are displayed in such a manner that the latest value can be compared with the control value. Note that the displayed ST levels may be the maximum ST levels or ST levels at a specific timing.

At least one of the manner of display of the ST level display region (e.g., either the radar charts or the bar graphs) or the type of displayed ST levels can be switched by the user at a suitable timing.

The function keys displayed in the function key region 30 are changed in the enhanced test screen 300. The following describes representative function keys. When an operation is made on the TIMI key 35, the control unit 120 displays a screen for making additional input or correction of the risk score (here, the TIMI risk score) (details will be described later).

When an operation is made on a report key 41, the control unit 120 ends the enhanced test at that point in time and causes the printer 119 to output a summary report (which will be described later) based on results of analysis processing performed heretofore.

When an operation is made on a 12-lead key 42, the control unit 120 ends the operations in the enhanced mode. Note that even after the operations in the enhanced mode are ended, real-time display of the standard 12-lead ECG is continued. Note that, in response to the operation made on the 12-lead key 42, the control unit 120 causes the display unit 118 to display a message confirming whether or not the user wants to end the enhanced test, and ends the enhanced test only when the user gives an explicit instruction to end the enhanced test. In this case, the summary report, which will be described later, is not output.

Referring back to FIG. 2B, in step S221, the control unit 120 determines whether or not a condition for executing analysis processing (ECG test) is satisfied, and upon determining that the condition is satisfied, executes step S223, otherwise executes step S225. Here, the condition for executing analysis processing may be a period of time elapsed from execution of the latest analysis processing, for example. It is recommended to perform an ECG test in every 5 to 10 minutes in the guideline, and accordingly, the period of time may be set to 5 to 10 minutes. However, it is thought that a long interval is set in the guideline assuming that a medical stuff performs manual recording. Therefore, in the present embodiment, analysis processing is executed at an interval (e.g., 30 seconds) shorter than the interval recommended in the guideline, to realize a more thorough ECG test.

Note that it is also possible to determine that a condition for executing analysis processing is satisfied, when an operation made on the recording button included in the operation unit 117 is detected. In this case, the control unit 120 executes analysis processing in response to the operation made on the recording button, in addition to the periodically executed analysis processing. Note that a configuration is also possible in which the user can set conditions for executing analysis processing in the enhanced test.

In step S223, the control unit 120 (analysis unit 1201) stores a copy of 12-lead ECG data of the latest predetermined period of time (e.g., 10 seconds), which is buffered in the RAM 124, in another region of the RAM 124 or the ROM 125 and then applies analysis processing. The ECG data is copied because the ECG data to which the analysis processing is applied may be used to output a summary report. The ECG data need not be copied in a case where data of the measured ECG is not deleted, such as a case where the buffer of the RAM 124 can hold 12-lead ECG data of several hours, for example.

The analysis processing applied in this step may be the same as the analysis processing started in step S203. Note that the analysis processing includes detection of ST levels of the type to be displayed, with respect to each lead.

Also, the control unit 120 generates composite lead data of the predetermined period of time by performing composite processing using the 12-lead ECG data of the predetermined period of time to which the analysis processing is applied, and stores the generated data in the RAM 124. Accordingly, the composite lead data is generated intermittently. Also, the control unit 120 (analysis unit 1201) applies analysis processing to the generated composite lead data and detects ST levels of the type to be displayed, with respect to each lead. Note that, in the present embodiment, only analysis processing that is necessary to detect the ST levels is applied to the composite lead data. However, analysis processing equivalent to the analysis processing applied to the standard 12-lead ECG data may also be applied to the composite lead data. In this case, the analysis processing may also be applied to ECG data of the 18 leads after the composite processing is performed.

Furthermore, the control unit 120 updates representative waveforms of the standard 12 leads, representative waveforms of the 6 composite leads, and the ST levels shown in the enhanced test screen 300, and then executes step S225. As described above, the displayed representative waveforms and the magnitude of ST displacement are updated every time analysis processing is executed.

In step S225, the control unit 120 determines whether or not an instruction to input the risk score is detected, and upon determining that the instruction is detected, executes step S227, otherwise executes step S231. The instruction to input the risk score may be an operation made on the TIMI key 35.

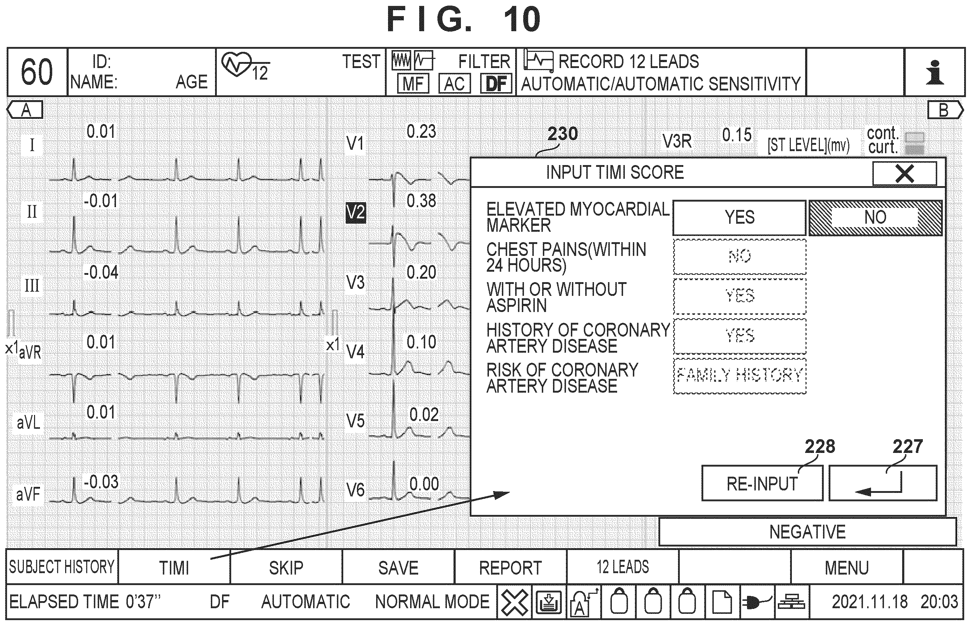

In step S227, the control unit 120 causes the display unit 118 to display a screen (re-input screen) for additional input or correction of the risk score. FIG. 10 shows an example of a TIMI score re-input screen 230. The TIMI score re-input screen 230 is similar to the TIMI score input screen 220, but as for items that have already been input, already input contents are grayed out, rather than options being displayed. FIG. 10 shows a state where items other than the myocardial marker have already been input.

When an operation is made on a re-input button 228, the control unit 120 changes display of the already input items to display including options other than the already input contents (display similar to that in the TIMI score input screen 220). However, the already input contents are displayed in the selected state among the options.

In step S229, the control unit 120 determines whether or not an instruction to end input or correction of the TIMI score is detected, and repeatedly executes step S227 unless it is determined that the instruction is detected. The instruction to end input or correction may be an operation made on the completion button 227.

When it is determined that an operation made on the completion button 227 is detected, the control unit 120 updates input contents of the TIMI score stored in the ROM 125 or the recording medium 116, for example, according to the states of the buttons included in the TIMI score re-input screen 230. Also, the control unit 120 ends display of the TIMI score re-input screen 230 and executes step S231.

In step S231, the control unit 120 determines whether or not a condition for ending the enhanced test is satisfied, and upon determining that the condition is satisfied, executes step S233, otherwise executes step S221 again. The condition for ending the enhanced test may be a period of time elapsed from the start of the enhanced test or an operation made on the report key 41 described above.

The period of time is set to 15 minutes or longer since the guideline recommends to perform measurement for at least 15 minutes. Although there is no particular limitation on the upper limit, the upper limit may be set to 1 hour, for example. Note that, when the enhanced test is ended based on the elapsed period of time, it is also possible to ask the user whether or not to extend the enhanced test to enable the user to extend the enhanced test.

Note that FIGS. 2A and 2B show the processing steps in the chronological order for the sake of convenience of making the flowchart, but some processing steps may be executed in parallel. For example, while the control unit is waiting for input of information to be finished in step S209, the automatic analysis started in step S203 is periodically executed, and the determination as to whether or not a finding for which ACS is suspected has been detected (S213) and the processing (S214, S215) performed according to the determination result are executed in parallel. Also, while additional input or correction of the risk score is performed in the loop including steps S227 and S228, the determination as to whether or not a condition for executing analysis processing (ECG test) is satisfied (S221) and the automatic analysis processing performed according to the determination result (S223) are executed in parallel.

When an operation is made on the report key 41, the control unit 120 may determine whether or not the number of times analysis processing has been executed in the enhanced test is less than a predetermined number of times (e.g., four times). Upon determining that the number of times is less than the predetermined number of times, the control unit 120 may cause the display unit 118 to display a message indicating that the ECG test has not been performed a sufficient number of times, and ask the user whether or not to end the enhanced test.

In step S233, the control unit 120 causes the printer 119 to output a summary report and ends the operations in the enhanced mode. Note that even after the operations in the enhanced mode are ended, real-time display of the 12-lead ECG is continued.

Next, the following describes the summary report. The summary report collectively presents information that is useful in making a diagnosis on the possibility of ACS in a single sheet among results of the ECG test (analysis processing) performed a plurality of times in the enhanced test. Conventionally, in order to make a diagnosis on the possibility of ACS from results of an ECG test performed a plurality of times, it has been necessary to compare individual reports output each time the ECG test is performed, and this is very cumbersome. The summary report output in this embodiment shows information useful in making a diagnosis on the possibility of ACS among the results of the ECG test performed a plurality of times on a single sheet, and therefore, it is unnecessary to compare a plurality of reports, and this is very convenient.

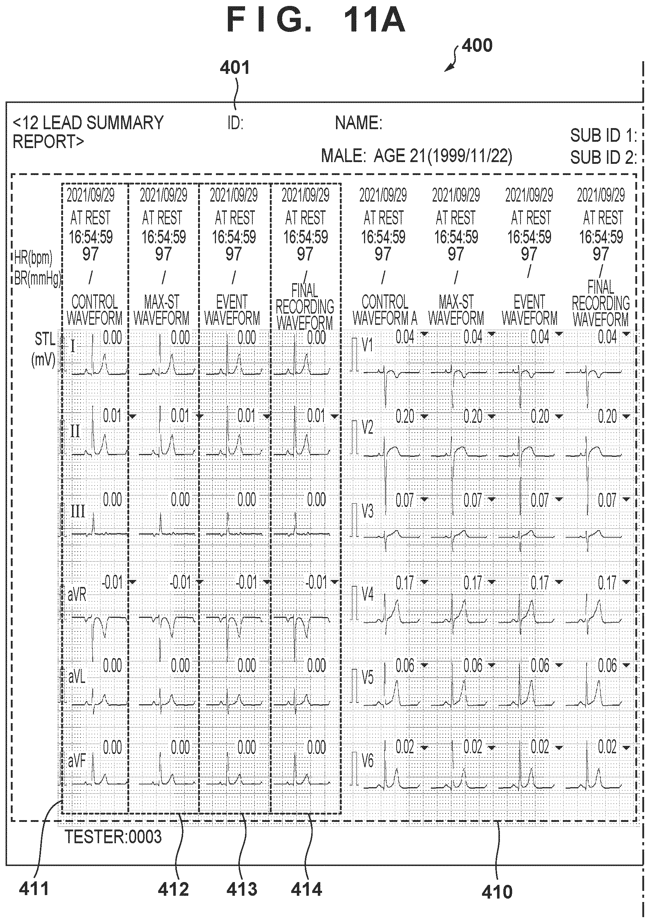

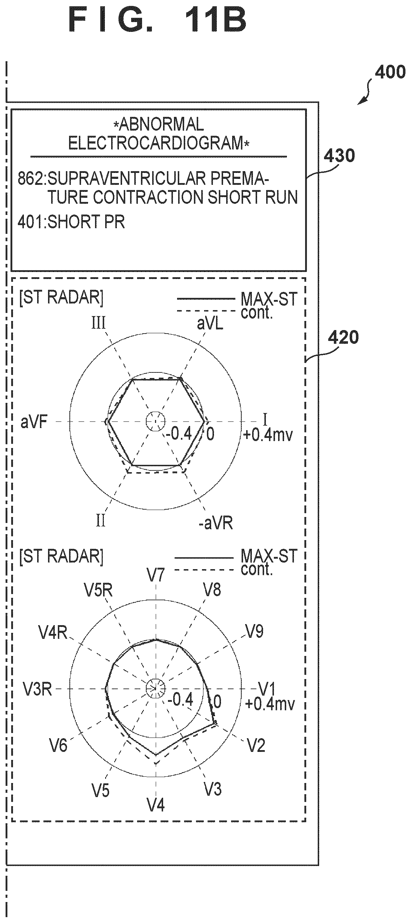

FIGS. 11A and 11B show an example of a summary report 400 output in step S233. The summary report 400 includes a subject information region 401, a waveform region 410 (first region), an ST level region 420 (second region), and a finding region 430 (third region).

The subject information region 401 is a region for presenting subject information including an ACS risk score (here, the TIMI risk score).

The waveform region 410 is a region for presenting a list of representative waveforms of the leads included in a standard 12-lead ECG of a predetermined period of time to which analysis processing has been applied, with respect to each of a plurality of ECG tests recorded in the report. In the present embodiment, the control unit 120 selects four ECG tests (analysis processing) to which the following conditions apply as the plurality of ECG tests recorded in the report, from ECG tests performed in the enhanced test.
(1) An ECG test performed immediately before or after the start of the enhanced test.
(2) An ECG test performed after the start of the enhanced test and in which the largest maximum ST level was detected.
(3) An ECG test performed after the start of the enhanced test and in which a finding of an abnormal ECG was detected.
(4) The last ECG test performed in the enhanced test.

Out of the conditions (1) to (4), (1) and (4) are exclusive. However, other combinations of the conditions may be satisfied by the same ECG test. Accordingly, the number of ECG tests to which the conditions (1) to (4) apply may be less than four. If the number of ECG tests to which the conditions (1) to (4) apply is less than four, an ECG test to which the condition (2) or (3) applies may be included twice or more in the summary report 400.

Then, with respect to the selected four ECG tests, the control unit 120 records representative waveforms 411 to 414 of each lead included in the standard 12-lead ECG of the predetermined period of time to which the analysis processing has been applied, in the waveform region 410.

The representative waveform 411 in the ECG test (1) is the control waveform described above. (1) and (4) are important to grasp changes in the ECG over time from the start to the end of the enhanced test. Also, (2) and (3) are both important in making a diagnosis on the possibility of ACS. As for (3), if findings indicating abnormality were detected in two or more ECG tests, an ECG test in which the most important finding determined in advance was detected is selected.

In this example, the number of ECG tests for which waveforms are recorded in the summary report 400 is four, but the number of ECG tests may also be set to three adopting the conditions (1), (2), and (4) or the conditions (1), (3), and (4). Alternatively, the number of ECG tests may be set to five or more adopting two or more ECG tests to which the condition (3) applies. When the number of ECG tests for which waveforms are recorded is increased, it becomes difficult to grasp small changes in the waveforms, and therefore, it is desirable to give consideration to visibility when the number of ECG tests is set to five or more.

The control unit 120 produces the summary report 400 such that the summary report 400 can be represented within a single sheet comprehensively with good visibility. Although the size of the sheet depends on the size of the printer 119, the control unit 120 produces the summary report 400 such that the summary report 400 can be shown on a sheet of A4 size or B4 size. The control unit 120 produces print data of the summary report 400 according to the size of the sheet and supplies the print data to the printer 119 to output the summary report 400.

It is desirable that the waveforms presented in the waveform region 410 are shown in the same scale as waveforms included in an ECG output from the printer 119 while the electrocardiograph is operating in the normal mode or shown in the same scale as far as possible. On the other hand, in order to help efficient diagnosis by eliminating the labor of comparing multiple sheets of report, it is necessary to collectively show the results of the plurality of ECG tests in a single sheet. Therefore, waveforms of the 6 composite leads are not included in the waveform region 410, and only waveforms of the standard 12 leads are presented. On the other hand, maximum ST levels are presented with respect to leads including 6 composite leads.

However, the control unit 120 may also cause the printer 119 to output waveforms of the standard 12 leads and the 6 composite leads in the ECG tests to which the conditions (1) to (4) described above apply, as a report other than the summary report 400 in response to an instruction from the user.

The ST level region 420 presents the maximum ST level of each lead. Similarly to the ST level display region 330 shown in FIG. 6, the ST level region 420 presents the maximum ST level of each lead by using separate radar charts for the 6 limb leads and the 12 chest leads including the composite leads. However, in the ST level region 420, maximum ST levels (cont) in the ECG test (1) used as the control waveforms and maximum ST levels detected in the ECG test (2) (maximum ST levels in the entire enhanced test) (MAX-ST) are presented in such a manner that the maximum ST levels (cont) and the maximum ST levels (MAX-ST) can be compared with each other.

A list of findings of abnormal ECGs detected in the analysis processing performed in the enhanced test is presented in the finding region 430 in descending order of the importance of the findings.

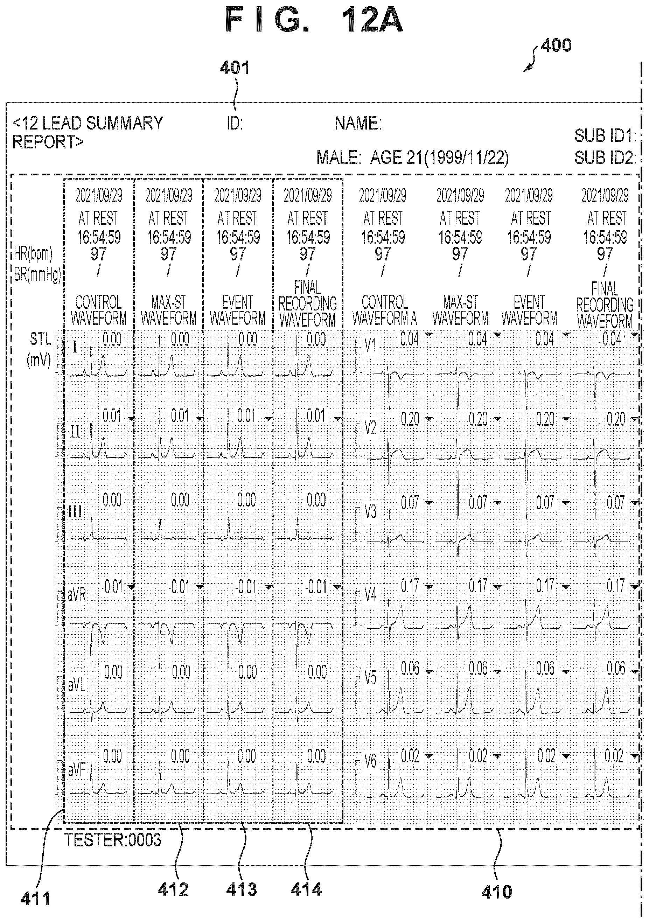

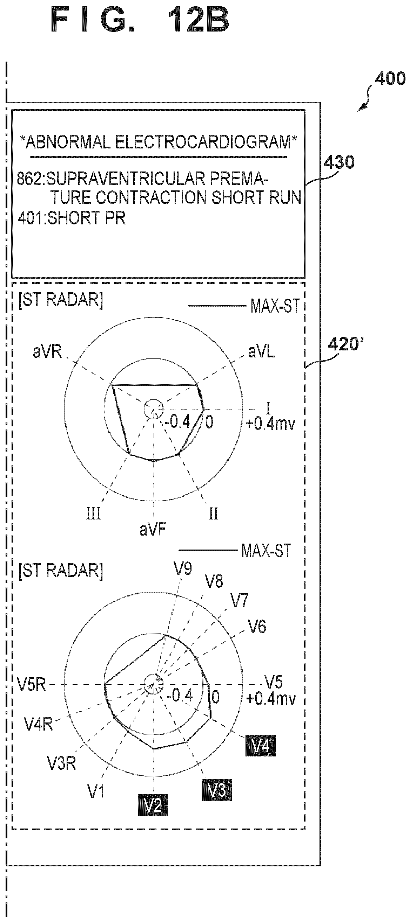

Note that similarly to the variations of the enhanced test screen 300, at least one of: the forms of the radar charts shown in the ST level region; or the types of plotted ST levels may be changed in the summary report 400. FIGS. 12A and 12B show a variation of the summary report 400 including an ST level region 420' in which the forms of radar charts and the types of plotted ST levels are changed.

In the variation shown in FIGS. 12A and 12B, arrangement of the axes of the radar charts is changed according to a positional relationship that simulates a positional relationship between the electrodes respectively corresponding to the leads on the body surface, as in the variations shown in FIGS. 7 and 8. Furthermore, the types of plotted ST levels are changed to only the maximum ST level detected in the enhanced test. Note that it is also possible to change only the arrangement of the axes or only the types of plotted ST levels.

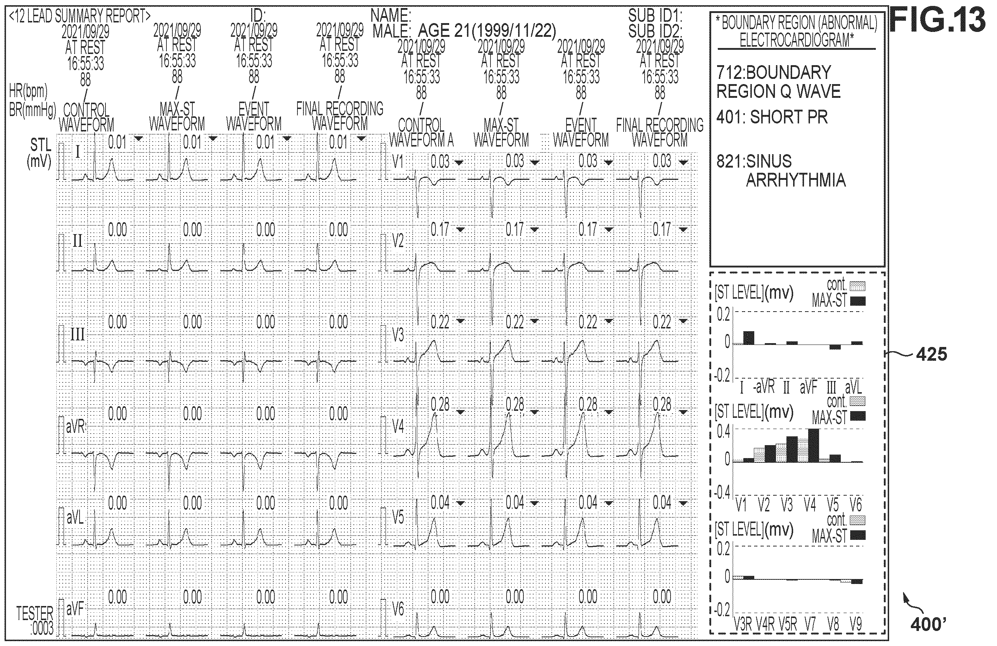

FIG. 13 shows an example of a summary report 400' including an ST level region 425 in which bar graphs 341a to 341c similar to those shown in FIG. 9 are used. ST levels are recorded in the ST level region 425 in the same manner as in the bar graphs 341a to 341c included in the enhanced test screen 300'. In the ST level region 425, maximum ST levels (cont) in the ECG test (1) used as the control waveforms and maximum ST levels detected in the ECG test (2) (maximum ST levels in the entire enhanced test) (MAX-ST) are presented in such a manner that the maximum ST levels (cont) and the maximum ST levels (MAX-ST) can be compared with each other. In the case where the bar graphs are used as well, the summary report may also be changed such that only the maximum ST levels detected in the enhanced test are presented.

The user can select the form of the ST level region and the types of plotted ST levels in the summary report. The control unit 120 causes the printer 119 to output the summary report including the ST level region in the form according to a setting set when step S233 is executed.

The electrocardiograph according to the present embodiment has the enhanced mode in which the electrocardiograph automatically executes an ECG test for evaluating the possibility of ACS without waiting for an explicit instruction from the user. In the enhanced mode, the electrocardiograph periodically performs the ECG test (analysis processing) while continuously executing measurement and real-time display of an ECG, and upon detecting a finding for which ACS is suspected, executes a more detailed enhanced test. Also, the electrocardiograph executes the enhanced test when an ACS risk score of the subject that is input is higher than or equal to a predetermined value.

As described above, the electrocardiograph according to the present embodiment determines whether or not ACS is suspected for the subject based on the analysis processing or the risk score even without a specific instruction from the user. Accordingly, the user can know whether or not a more detailed ECG test should be carried out on the subject, without the need to instruct the electrocardiograph to execute a test at predetermined intervals or request a doctor to read a report of a test result output from the electrocardiograph.

In the enhanced test as well, the electrocardiograph periodically performs an ECG test (analysis processing) while continuously executing measurement and real-time display of an ECG. In the enhanced test, the electrocardiograph generates a right chest lead and posterior leads, of which recording is recommended in the guideline, through composite processing, and detects an ST level of each lead including the standard 12 leads. Also, when the enhanced test is finished, the electrocardiograph outputs a summary report collectively showing results of a plurality of ECG tests that are useful in making a diagnosis on the possibility of ACS on a single sheet, among ECG tests periodically performed in the enhanced test.

It is conceivable that sufficient human resources cannot be used for ECG tests because various treatments need to be performed in parallel on a subject who has been taken to an emergency room due to having a pain in the chest. Accordingly, an additional ECG test or evaluation like that recommended in the guideline may not be sufficiently performed on a subject for whom it is not possible to make a diagnosis on ACS based on an initial ECG. A situation in which a correct diagnosis cannot be made on the possibility of ACS for the subject due to the ECG test not being sufficiently performed over time should be avoided for the subject and medical stuffs.

The electrocardiograph according to the present embodiment automatically evaluates the risk of ACS without the user being involved, and automatically executes a detailed ECG test recommended in the guideline for a subject for whom the possibility of ACS cannot be denied. Furthermore, the electrocardiograph outputs a summary report collectively showing results of a plurality of ECG tests that are useful for a doctor when finally making a diagnosis on the possibility of ACS, and this eliminates the labor of the user managing or comparing a plurality of reports, and contributes to an appropriate diagnosis made by the doctor. As described above, with the electrocardiograph according to the present embodiment, it is possible to execute an appropriate ECG test particularly for a subject for whom it is not possible to make a diagnosis of ACS based on the first ECG but the possibility of ACS cannot be denied, while reducing a burden on medical stuffs.

### (Other embodiments)

The present invention can be implemented by a program that causes a computer to function as an ECG analysis device described in the above embodiment. The present invention is not limited to the above embodiment, and various changes and modifications can be made within the spirit and scope of the present invention. Therefore, to apprise the public of the scope of the present invention, the following claims are made.

This application claims priority from Japanese Patent Application No. 2022-70086 filed April 21, 2022, which is hereby incorporated by reference herein.

## Claims

1. An electrocardiograph comprising:
obtaining means for continuously obtaining an electrocardiogram of a subject;
display means for continuously displaying the electrocardiogram on a display device;
analysis means for automatically applying analysis processing to the electrocardiogram in parallel with display of the electrocardiogram by the display means; and
determination means for determining whether or not information obtained through the analysis processing satisfies a condition determined in advance regarding a possibility of acute coronary syndromes (ACS),
the electrocardiograph further comprising control means for controlling the electrocardiograph to execute an enhanced test including periodical execution of an electrocardiogram test when it is determined by the determination means that the information obtained through the analysis processing satisfies the condition regarding the possibility of ACS before an ending condition determined in advance is satisfied.

2. The electrocardiograph according to claim 1,
wherein the determination means further determines whether or not an ACS risk score regarding the subject is higher than or equal to a threshold determined in advance, and
the control means controls the electrocardiograph to execute the enhanced test when it is determined by the determination means that the ACS risk score regarding the subject is higher than or equal to the threshold determined in advance, before the ending condition is satisfied.

3. The electrocardiograph according to claim 2,
wherein the ACS risk score is a TIMI risk score or a GRACE risk score.

4. The electrocardiograph according to any one of claims 1 to 3,
wherein the analysis means periodically applies the analysis processing to the electrocardiogram until the ending condition is satisfied or it is determined by the determination means that the information obtained through the analysis processing satisfies the condition regarding the possibility of ACS.

5. The electrocardiograph according to any one of claims 1 to 3,
wherein the control means suspends execution of the enhanced test until an instruction given by a user to execute the enhanced test is detected.

6. The electrocardiograph according to any one of claims 1 to 3,
wherein the information obtained through the analysis processing is a finding, and
the control means displays, on the display device, a finding for which it is determined by the determination means that the condition is satisfied.

7. The electrocardiograph according to any one of claims 1 to 3,
wherein the enhanced test includes, in parallel with continuous display of the electrocardiogram by the display means:
periodically applying analysis processing to the electrocardiogram by the analysis means;
generating data of a lead that is not included in the electrocardiogram by performing composite processing based on the electrocardiogram by the control means; and
detecting an ST level for each lead included in the electrocardiogram and each lead generated through the composite processing by the control means.

8. The electrocardiograph according to claim 7,
wherein the electrocardiogram is a standard 12-lead electrocardiogram, and the lead that is not included in the electrocardiogram includes a right chest lead and a posterior lead.

9. The electrocardiograph according to claim 7,
wherein the lead that is not included in the electrocardiogram includes lead V4R and leads V7 to V9.

10. The electrocardiograph according to claim 7,
wherein the ST level is a representative value of an ST level based on an amplitude or a timing.

11. The electrocardiograph according to claim 10,
wherein the representative value of the ST level based on the amplitude is a maximum ST level.

12. The electrocardiograph according to claim 10,
wherein the representative value of the ST level based on the timing is an ST level at a timing when a predetermined period has elapsed from the start of an ST segment.

13. The electrocardiograph according to any one of claims 1 to 3,
wherein, when a condition for ending the enhanced test is satisfied, the control means causes an output device to output a report collectively showing a predetermined number of analysis processing results among results of analysis processing executed in the enhanced test.

14. The electrocardiograph according to claim 13,
wherein the predetermined number of analysis processing results include a result of analysis processing executed immediately before or after the start of the enhanced test and a result of last analysis processing executed in the enhanced test.

15. The electrocardiograph according to claim 13,
wherein the predetermined number of analysis processing results include at least one of a result of analysis processing in which a maximum ST level is detected in the enhanced test or a result of analysis processing in which an abnormal finding is detected.

16. The electrocardiograph according to claim 13,
wherein the results of analysis processing include a representative waveform and a maximum ST level of each lead.

17. A method for controlling an electrocardiograph comprising:
continuously obtaining an electrocardiogram of a subject;
continuously displaying the electrocardiogram on a display device;
in parallel with continuous display of the electrocardiogram,
automatically applying analysis processing to the electrocardiogram;
determining whether or not information obtained through the analysis processing satisfies a condition determined in advance regarding a possibility of acute coronary syndromes (ACS); and
when it is determined that the information obtained through the analysis processing satisfies the condition regarding the possibility of ACS before an ending condition determined in advance is satisfied, controlling the electrocardiograph to execute an enhanced test including periodical execution of an electrocardiogram test.

18. A program for causing a computer included in an electrocardiograph to function as each means included in the electrocardiograph according to claim 1.
